Europäisches Patentamt

European Patent Office

Office européen des brevets

(11) Publication number: **0 253 924**
**A1**

(12) **EUROPEAN PATENT APPLICATION**

(21) Application number: 86201283.8

(22) Date of filing: 21.07.86

(51) Int. Cl.³: **B 01 J 23/74**
**//B01J23/89**

(43) Date of publication of application:
27.01.88 Bulletin 88/4

(84) Designated Contracting States:
AT BE DE FR GB IT NL SE

(71) Applicant: SHELL INTERNATIONALE RESEARCH
MAATSCHAPPIJ B.V.
Carel van Bylandtlaan 30
NL-2596 HR Den Haag(NL)

(72) Inventor: Kobylinski, Thaddeus P.
Road 1
prospect Pennsylvania 16052(US)

(72) Inventor: Kibby, Charles Lee
846 Cliston CT
Benicia California 94510(US)

(72) Inventor: Pannel, Richard Byron
5252 Hagerstown Avenue
Baton Rouge Louisiana 70817(US)

(72) Inventor: Eddy, Elizabeth Leigh
3093 Camberly Drive
Gibsonia Pennsylvania 15044(US)

(74) Representative: Aalbers, Onno et al,
P.O. Box 302
NL-2501 CH The Hague(NL)

(54) Synthesis gas conversion using ROR-activated catalyst.

㊼ Process for the preparation of a supported cobalt or nickel catalyst by depositing a cobalt or nickel precursor on a support by impregnation or precipitation, and activating said catalyst by subjecting it to (i) reduction with hydrogen, (ii) oxidation with an oxygen containing gas, and (iii) reduction with hydrogen at a temperature between 100 and 450 °C. The invention further relates to the catalysts thus prepared, as well as to the use of the catalysts in the conversion of synthesis gas to liquid hydro-carbons.

EP 0 253 924 A1

# SYNTHESIS GAS CONVERSION USING ROR-ACTIVATED CATALYST

The invention relates to a process for the preparation of a supported cobalt or nickel catalyst that has been subjected to an activation treatment to provide improved activity and selectivity, to the catalyst thus prepared, and to the use of the catalyst in the conversion of synthesis gas to liquid hydrocarbons. The invention particularly relates to activated catalysts prepared by impregnating a refractory metal oxide support with a cobalt-carbonyl compound.

U.S. Patent No. 4,088,671 describes a ruthenium-promoted cobalt catalyst on a support, such as alumina or kieselguhr, for the preparation of hydrocarbons from CO and hydrogen. As ruthenium is expensive, the patent indicates that it is preferred to employ ruthenium in the minimum amount necessary to achieve the desired result. Attempts have been made to utilize unpromoted cobalt catalysts for the synthesis of hydrocarbons from synthesis gas. However, unpromoted cobalt often had poor selectivity and requires high metal loadings to provide desirable activity.

It has now been found that synthesis gas comprising hydrogen and carbon monoxide can be selectively converted under synthesis gas conversion conditions to liquid hydrocarbons with a catalyst prepared by subjecting a supported cobalt or nickel catalyst to an activation procedure comprising the steps, in sequence, of (A) reduction in hydrogen, (B) oxidation in an oxygen-containing gas, and (C) reduction in hydrogen, herein termed "ROR activation", the activation procedure being conducted at a temperature below 450 °C. The activation procedure provides supported cobalt and nickel catalysts with improved reaction rates regardless of whether the catalyst is prepared by impregnation of a support with cobalt or nickel, or by precipitation of cobalt or nickel onto the support. Moreover, the activation procedure can significantly improve the activity of promoted, supported cobalt and nickel catalysts, wherein promoters, such as ruthenium and lanthana, have been previously added to improve activity.

The invention therefore relates to a process for the preparation of an activated, supported catalyst comprising depositing a cobalt or nickel precursor on a refractory metal oxide support by impregnation or precipitation to form a supported catalyst, and activating said supported catalyst by subjecting said supported catalyst to the steps, in sequence, of (i) reduction in hydrogen gas, (ii) oxidation in an oxygen-containing gas and (iii) reduction in hydrogen gas, steps (i), (ii) and (iii) being conducted at a temperature of from 100° to 450 °C.

The invention especially relates to the above process in which the cobalt precursor is a cobalt carbonyl compound as the activated, cobalt carbonyl-impregnated catalyst provides very high reaction rates at moderate metal loadings, while at the same time, providing high selectivity, even without the use of the usual promoters. The unpromoted cobalt carbonyl catalyst shows an activity for synthesis gas conversion nearly comparable to conventional ruthenium-promoted catalysts made by impregnating cobalt nitrate on alumina, but without the ROR activation procedure of the present invention. Moreover, as will be hereinafter demonstrated, by subjecting the cobalt carbonyl impregnated catalyst to ROR activation, the resultant unpromoted catalyst produces as much $C_5$+ hydrocarbon product as do such ruthenium-promoted cobalt catalysts which have been subjected to ROR activation.

The supported cobalt and nickel catalysts may be prepared by any suitable procedure, whether precipitation of the cobalt or nickel precursor onto a refractory oxide support or impregnation of a refractory oxide support using an aqueous or non-aqueous impregnation solution of the nickel or cobalt precursor. In the activated, supported catalyst the cobalt or nickel is distributed as small crystallites upon the support.

Cobalt carbonyl catalysts are preferably prepared by impregnating a support comprising a refractory oxide formed of alumina,

- 3 -

in particular, gamma-alumina, eta-alumina or mixtures thereof, or silica with a cobalt carbonyl. Any suitable cobalt carbonyl compound may be employed, including dicobalt octacarbonyl, $Co_2(CO)_8$, tetra-cobalt dodecylcarbonyl, $Co_4(CO)_{12}$, or the like.

The catalyst may contain from 1 to 30 weight per cent cobalt based upon total catalyst weight, preferably from 3 to 20 weight per cent cobalt, with from 5 to 15 weight per cent cobalt being especially preferred. If nickel is the metal of choice, the catalyst may contain from 1 to 50 weight per cent nickel based upon total catalyst weight, preferably from 3 to 35 weight per cent nickel, with from 10 to 20 weight per cent nickel being especially preferred.

The refractory metal oxide support can be alumina or silica. Alumina is preferred, and the alumina is preferably a gamma or eta alumina. Likewise, extruded gamma or eta alumina can be used. A suitable support of the present invention is characterized as having low acidity, a high surface area and high purity. The expression "low acidity" as used in the present application means that the support has a Brönsted activity with $H_o < 1.5$ which is less than 5 micromol per gram or about $10^{16}$ acid sites per square metre of surface area. The low acidity of the support is required in order to enable the catalyst to provide a higher molecular weight hydrocarbon product.

The surface area of the support of the present invention is at least 40 or 50 square metres per gram but is not so great as to become unduly microporous so as to permit reactant materials to enter the interstices of the catalyst. A suitable surface area is from 40 to 250, preferably from 150 to 225 square metres per gram.

As indicated, the catalyst support of the present invention should be of high purity. When the catalyst support is alumina, the expression "high purity" means that the catalyst contains negligible amounts of sulphur, silicon, phosphorous or other material having a deleterious effect on the metal dispersion or the production of high molecular weight hydrocarbon products. When a silica support

- 4 -

is used, the expression "high purity" means that the catalyst contains negligible amounts of sulphur, aluminium, phosphorous or other material having a deleterious effect on the metal dispersion or the production of high molecular weight hydrocarbon products. For sulphur, the impurity levels should be below 0.1 weight per cent, preferably below 0.02 weight per cent, and especially below 0.01 weight per cent. For impurities creating acid sites, less than 5 micromol per gram should be present (about 0.01-0.1 weight per cent depending on molecular weight). The deleterious effect of acidity is isomerization and cracking of intermediate olefins, removing them from chain growth and producing a low molecular weight product.

Ruthenium may be included in the catalyst as a promoter. The amount of ruthenium can be from 0.01 to 0.50 weight per cent, preferably from 0.05 to 0.25 weight per cent based upon total catalyst weight. This helds also for the cobalt carbonyl catalyst, although the unpromoted cobalt carbonyl on alumina or silica catalyst is already extremely active for conversion of synthesis gas and highly selective for the production of $C_5+$ hydrocarbons.

The catalysts of the present invention may additionally contain from 0.1 to 5 weight per cent, preferably from 0.1 to 2 weight per cent of a suitable promoter metal oxide, such as $La_2O_3$, $MnO_2$, or a Group IIIB or IVB metal oxide. Oxides of the lanthanides and actinides are preferred. Thus, suitable metal oxides include, for example, $Sc_2O_3$, $Y_2O_3$, $Ac_2O_3$, $Pr_2O_3$, $PrO_2$, $Nd_2O_3$, $Sm_2O_3$, $Eu_2O_3$, $Gd_2O_3$, $Tb_2O_3$, $Tb_4O_7$, $Dy_2O_3$, $Ho_2O_3$, $Er_2O_3$, $Tm_2O_3$, $Yb_2O_3$, $Lu_2O_3$, $UO_2$, $UO_3$, $U_3O_8$, and the like. Especially preferred metal oxides for inclusion in the catalyst of the present invention include $La_2O_3$, $CeO_2$, $ZrO_2$, $TiO_2$, $HfO_2$, $ThO_2$, and unseparated rare earth oxides mixtures high in lanthanum, praseodymium, and neodymium. Additional preferred promoters are MgO and $MnO_2$.

The ROR activation procedure of the present invention may be used to improve activity of the supported catalyst regardless of the method used to deposit the catalytic metals on the support. Thus, any technique well known to those having ordinary skill in the art to distend the catalytic metals in a uniform thin layer on the catalyst support is suitable here. For example, the cobalt or nickel can be deposited onto the support material by the technique of minimum excess solution from an aqueous solution of a suitable cobalt or nickel containing compound, such as a nitrate, chloride, or acetate; or the cobalt or nickel can be precipitated from an aqueous solution onto a support by technique is illustrated in U.S. Patent No. 4,088,671 the disclosure of which is hereby incorporated by reference.

A preferred method employed to deposit the catalytic metals onto the support involves an impregnation technique using non-aqueous, organic impregnation solutions of soluble cobalt or nickel salt and, if desired, a soluble promoter metal salt, e.g., a ruthenium or lanthanum salt, in order to achieve the desired metal loading and distribution.

Initially, the support, such as alumina, can be treated by oxidative calcination of the gamma and/or eta-alumina at a temperature in the range of from 450° to 900 °C, preferably from 600° to 750 °C to remove water from the micropores of the support.

Meanwhile, a non-aqueous organic solvent solution of a cobalt or nickel salt, and if desired, non-aqueous organic solvent solutions of ruthenium, lanthanum, and/or manganese salts, for example, are prepared. Any suitable ruthenium salt, such as ruthenium nitrate, chloride, acetate or the like can be used. In addition, any suitable promoter metal, e.g., a lanthanum salt, such as lanthanum nitrate or lanthanum acetate or manganese salt, such as manganese nitrate, or the like can be employed.

The non-aqueous organic solvent is a non-acidic liquid which is formed from moieties selected from the group consisting of carbon, oxygen, hydrogen and nitrogen, and possesses a relative volatility of at least 0.1. The expression "relative volatility" as used in the present application is defined as the ratio of the vapour pressure of the solvent to the vapour pressure of acetone, as reference, when measured at 25 °C.

Suitable solvents include, for example, ketones, such as acetone, butanone (methyl ethyl ketone); the lower alcohols, e.g., methanol, ethanol, propanol and the like; amides, such as dimethyl formamide; amines, such as butylamine; ethers, such as diethylether and tetrahydrofuran; hydrocarbons, such as pentane and hexane; and mixtures of the foregoing solvents. The preferred solvents of the present invention are acetone, for cobalt nitrate, or tetrahydrofuran.

Suitable solvents for cobalt carbonyls, for instance dicobalt octacarbonyl, are hydrocarbons, such as pentane, hexane and toluene; ethers, such as tetrahydrofuran, or mixtures of the foregoing solvents.

The amount of solvent utilized is an amount that is at least equivalent to the pore volume of the alumina utilized, but not greater than five times the alumina pore volume. For example, a commercially available gamma-alumina useful in the present invention has a pore volume of between 0.2 to 0.7 cubic centimetres pore volume per gram of alumina.

Suitable cobalt salts include, for example, cobalt nitrate, cobalt acetate, cobalt carbonyl, cobalt acetylacetonate, or the like. Suitable nickel salts include nickel nitrate, nickel acetate, nickel carbonyl, nickel acetylacetonate, or the like. Likewise, any suitable ruthenium salt, such as ruthenium nitrate, chloride, acetate or the like can be used. Ruthenium acetylacetonate is preferred. In addition, any suitable promoter metal, e.g., a lanthanum salt, such as lanthanum nitrate, lanthanum acetate or the like can be employed. In general, any metal salt which will not introduce acidity or have a poisonous effect on the catalyst can be utilized.

The calcined alumina support is then impregnated in a dehydrated state with the non-aqueous, organic solvent solution of the metal salts. Thus, the calcined alumina should not be unduly exposed to the atmospheric humidity so as to become rehydrated.

Any suitable impregnation technique can be employed including techniques well known to those skilled in the art so as to distend the catalytic metals in a uniform thin layer on the catalyst support. For example, the cobalt or nickel along with the oxide promoter can be deposited on the support material by the "incipient wetness" technique. Such technique is well known and requires that the volume of impregnating solution be predetermined so as to provide the minimum volume which will just wet the entire surface of the support, with no excess liquid. Alternatively, the excess solution technique can be utilized if desired. If the excess solution technique is utilized, then the excess solvent present, e.g., acetone, is merely removed by evaporation. Thus, the impregnation solution can be added in excess, namely, up to five times the pore volume of the support, or can be added using just enough solution to fill the pore volume of the support.

Next, the impregnation solution and alumina are stirred while evaporating the solvent at a temperature of from 25° to 50 °C until "dryness".

The impregnated catalyst is slowly dried at a temperature of from 110° to 120 °C for a period of about 1 hour so as to spread the metals over the entire support. The drying step is conducted at a very slow rate in air.

The dried catalyst may be reduced directly in hydrogen or it may be calcined first. In the case of impregnation with cobalt nitrate, direct reduction can yield a higher cobalt metal dispersion and synthesis activity, but reduction of nitrates is difficult to control and calcination before reduction is safer for large scale preparations. Also, a single calcination step to decompose nitrates is simpler if multiple impregnations are needed to provide the desired metal loading. Reduction in hydrogen requires a prior purge with inert gas, a subsequent purge with inert gas and a passivation step in addition to the reduction itself, as described later as part of the ROR activation. However, impregnation of cobalt carbonyl must be carried out in a dry, oxygen-free atmosphere and it must be reduced directly, then passivated, if the benefits of its lower oxidation state are to be maintained.

The dried catalyst is calcined by heating slowly in flowing air, for example 10 cc/gram/minute, to a temperature in the range of from 200° to 400 °C, preferably from 250° to 300 °C,.that is sufficient to decompose the metal salts and fix the metals. The aforesaid drying and calcination steps can be done separately or can be combined. However, calcination should be conducted by using a slow heating rate of, for example, 0.5° to 3 °C per minute, preferably from 0.5° to 1 °C per minute and the catalyst should be held at the maximum temperature for a period of 1 to 20 hours, preferably for 2 hours.

The foregoing impregnation steps are repeated with additional impregnation solutions in order to obtain the desired metal loading. Ruthenium and other promoter metal oxides are conveniently added together with cobalt or nickel, but they may be added in other impregnation steps, separately or in combination, either before, after, or between impregnations of cobalt or nickel.

In the case of the cobalt carbonyl catalyst, it is preferred that first the impregnation steps are performed with impregnation solutions of the promoter precursors in order to obtain the desired promoter metal loading. The calcined support is then impregnated in a dehydrated state with the non-aqueous, organic solvent solution of the cobalt carbonyl. Thus, the calcined support should not be unduly exposed to atmospheric humidity so as to become rehydrated. The impregnation technique used for dicobalt octacarbonyl may be the same as described above for the promoter salts, except that an oxygen-free water-free atmosphere, such as argon, helium or nitrogen, must be employed. Preferably, an incipient wetness technique is used so that the solvent may be removed easily. If more than one impregnation is required to achieve the desired metal loading, the cobalt carbonyl deposited in the first step must be decomposed by a reduction-reoxidation treatment, which are the first two steps of the "ROR"-activation treatment of the present invention.

After the last impregnation sequence, the loaded catalyst support is then subjected to the ROR activation treatment of the present invention. The ROR activation treatment of the present invention must be conducted at a temperature below 450 °C in order to achieve the desired increase in activity and selectivity of the cobalt- or nickel-impregnated catalyst. Temperatures of 450 °C or above reduce liquid hydrocarbon selectivity of the cobalt- or nickel-impregnated catalyst. Suitable ROR activation temperatures are below 450 °C, preferably below 400 °C. Thus, ranges of 100° or 150° to 450 °C, preferably 250° to 400 °C are suitable for reduction and oxidation steps. The activation steps are conducted while heating at a rate of from 0.1° to 5 °C, preferably from 0.1° to 2 °C.

The impregnated catalyst is preferably slowly reduced in the presence of hydrogen. If the catalyst has been calcined after each impregnation, to decompose nitrates or other salts, then the reduction may be performed in one step with heating in a single temperature ramp (e.g., 1 °C/min.) to the maximum temperature and held at that temperature, from 250° or 300° to 450 °C, preferably from 350° to 400 °C, for a hold time of 6 to 65 hours, preferably from 16 to 24 hours. If nitrates are still present, the reduction is best conducted in two steps wherein the first reduction heating step is carried out at a slow heating rate of no more than 5 °C per minute, preferably from 0.1° to 1 °C per minute up to a maximum hold temperature of 200° to 300 °C, preferably 200° to 250 °C, for a hold time of from 6 to 24 hours, preferably from 16 to 24 hours under ambient pressure conditions. In the second reduction heating step, the catalyst can be heated at from 0.5° to 3 °C per minute, preferably from 0.1° to 1 °C per minute to a maximum hold temperature of from 250° or 300° up to 450 °C, preferably from 350° to 400 °C for a hold time of 6 to 65 hours, preferably from 16 to 24 hours. Although pure hydrogen can be employed for these reduction steps, a mixture of hydrogen and nitrogen can be utilized in order to slowly reduce the catalyst. For example, the reduction can be conducted initially using a gaseous mixture comprising 5% hydrogen and 95% nitrogen, and thereafter, the concentration of hydrogen can be gradually increased until pure hydrogen is obtained so as to slowly reduce the catalyst.

Such slow reduction is particularly desirable when the metal salts utilized in the impregnation step are nitrates so as to avoid the dangers involved with an exothermic reaction in which nitrates are reduced. Thus, the slow reduction may involve the use of a mixture of hydrogen and nitrogen at 100 °C for about one hour; increasing the temperature 0.5 °C per minute until a temperature of 200 °C; holding that temperature for approximately 30 minutes; and then increasing the temperature 1 °C per minute until a temperature of 350 °C is reached and then continuing the reduction for approximately 16 hours. Reduction should be conducted slowly enough, especially in the case of cobalt carbonyl in order that excessive volatilization does not occur, and the flow of the reducing gas maintained high enough to maintain the partial pressure of water in the offgas below 1 per cent, so as to avoid excessive steaming of the exit end of the catalyst bed. Before and after all reductions, the catalyst must be purged in an inert gas such as nitrogen, argon or helium.

The reduced catalyst is passivated at ambient temperature (25-35 °C) by flowing diluted air over the catalyst slowly enough so that a controlled exotherm passes through the catalyst bed. After passivation, the catalyst is heated slowly in diluted air to a temperature of from 300° to 350 °C in the same manner as previously described in connection with calcination of the catalyst.

Next, the oxidized catalyst is then slowly reduced in the presence of hydrogen in the same manner as previously described in connection with reduction of the impregnated catalyst. Since nitrates are no longer present, this reduction may be accomplished in a single temperature ramp and held, as described above for reduction of calcined catalysts.

The composite catalyst of the present invention has an average particle diameter, which depends upon the type of reactor to be utilized, of from 0.01 to 6 millimetres; preferably from 1 to 6 millimetres for a fixed bed; and preferably from 0.01 to 0.11 millimetres being preferred for a reactor with the catalyst suspended by gas, liquid, or gas-liquid media (e.g., fluidized beds, slurries, or ebullating beds).

The charge stock used in the process for the preparation of liquid hydrocarbons is a mixture of CO and hydrogen. Any suitable source of the CO and hydrogen can be used. The charge stock can be obtained, for example, by (i) the oxidation of coal or other forms of carbon with scrubbing or other forms of purification to yield the desired mixture of CO and $H_2$ or (ii) the reforming of natural gas. $CO_2$ is not a desirable component of the charge stocks for use in the process of this invention, but it may be present as a diluent gas. Sulphur compounds in any form are deleterious to the life of the catalyst and should be removed from the CO-$H_2$ mixture and from any diluent gases.

The reaction temperature is suitably from 160° to 350 °C, preferably from 175° to 275 °C, and most preferably from 185° to 250 °C. The total pressure is, for example, from 1 to 100 atmospheres, preferably from 3 to 35 atmospheres, and most preferably from 10 to 20 atmospheres. Surprisingly, it has been found that the use of pressures of at least 3.4 atmospheres using the low ruthenium catalysts of the present invention results in activities greater than that achievable with larger quantities of ruthenium at the same pressure.

The gaseous hourly space velocity based upon the total amount of feed is less than 20,000 volumes of gas per volume of catalyst per hour, preferably from 100 to 5000 v/v/hour, with from 1000 to 2500 v/v/hour being especially preferred. If desired, pure synthesis gas can be employed or, alternatively, an inert diluent, such as nitrogen, $CO_2$, methane, steam or the like can be added. As used herein, the expression "inert diluent" indicates that the diluent is non-reactive under the reaction conditions herein disclosed or is a normal reaction product.

The synthesis gas reaction using the catalysts of this invention can occur in a fixed, fluid or moving bed type of operation.

The invention will be further described with reference to the following experimental work. All percentages will be in terms of weight per cent unless otherwise indicated.

Example 1

The preparation of the catalyst of the present invention is exemplified by the following description.

A catalyst (denoted "catalyst A") was prepared by impregnating 22.002 grams of a gamma-alumina (Ketjen EC commercially availably from Akzo Chemie) with 8.700 grams of dicobalt octacarbonyl in tetrahydrofuran in an oxygen-free atmosphere. The alumina was treated with acetone and calcined at 300 °C prior to impregnation. The catalyst was loaded into a reactor in a glove-box. Initially, the catalyst was activated by heating at 5 °C per minute in 1680 cubic centimetres per gram per hour of hydrogen to a temperature of 185 °C at which temperature the catalyst was held for one hour. The reduced weight of the catalyst was 12 weight per cent cobalt and 88 weight per cent alumina.

The catalyst was subjected to a two-day synthesis run in which the catalyst was contacted with hydrogen and carbon monoxide at a ratio of 1.85 at a temperature of 185 °C under a pressure of one bar at a synthesis gas flow rate of 1680 cubic centimetres per gram of catalyst per hour on the first day, and thereafter subjecting the catalyst to a synthesis run on the second day under the same conditions with the exception of a temperature of 195 °C and a hydrogen to carbon monoxide ratio of 1.5. Thereafter, the catalyst was purged in hydrogen at 185 °C, and then heated at a rate of 1 °C per minute to a temperature of 350 °C and held at such temperature for a period of one hour (treatment to this point denoted "R350"). The catalyst was then subjected to another two days of synthesis at 185 °C and 195 °C, respectively, under the conditions previously indicated, and then purged in hydrogen, and cooled in nitrogen to room temperature. While under room temperature conditions, the catalyst was dosed with 6 pulses of an air/nitrogen mixture. Next, oxidation of the catalyst was conducted in flowing air by heating at a temperature of 1 °C per minute until the catalyst reached 300 °C where it was held for a period of five and one-half hours. The catalyst was then purged in nitrogen and cooled. Finally, the catalyst was reduced once again by heating at a rate of 1 °C per minute in hydrogen until a temperature of 350 °C was reached and then holding at that temperature for five and one-half hours (treatment to this point denoted "ROR").

For comparative purposes, a catalyst containing 20 weight per cent cobalt, 0.5 weight per cent ruthenium and 1.0 weight per cent $La_2O_3$ (catalyst B) prepared by impregnating gamma-alumina with cobalt nitrate rather than cobalt carbonyl, and a third catalyst containing 25 weight per cent cobalt, 0.5 weight per cent ruthenium and one weight per cent lanthanum oxide (catalyst C) also impregnated using cobalt nitrate, rather than cobalt carbonyl, were tested at the same time and thus under the identical conditions of the aforesaid cobalt carbonyl catalyst (catalyst A).

The results of the foregoing tests made at 185 °C and a hydrogen to carbon monoxide ratio of 1.85 are set forth in Table I, below, while the results of tests made at 195 °C at a hydrogen to carbon monoxide ratio of 1.5 are set forth in Table II, below:

### TABLE I

| Catalyst | CO Conversion Rate (cc/gram Co/hour) | | $C_5+$ Liquid (Wt. %) | |
|---|---|---|---|---|
| | R350 | ROR | R350 | ROR |
| A | 1230 | 1485 | 73 | 81 |
| B | 790 | 1190 | 80 | 79 |
| C | 690 | 960 | 81 | -- |

The results set forth in Table I demonstrate that the ROR treatment of the cobalt carbonyl catalyst (catalyst A) produces a catalyst having an activity (1485 cubic centimetres per gram of cobalt per hour), which is much greater than that of promoted catalysts (790 cubic centimetres per gram of cobalt per hour for catalyst B and 690 cubic centimetres per gram of cobalt per hour for catalyst C). Additionally, the activated cobalt carbonyl

- 14 -

catalyst A has a selectivity for the production of $C_5$+ hydrocarbon liquid comparable to the ruthenium-promoted cobalt catalyst.

TABLE II

| Catalyst | CO Conversion Rate (cc/gram Co/hour) | | $C_5$+ Liquid (Wt. %) | |
|---|---|---|---|---|
| | R350 | ROR | R350 | ROR |
| A | 1525 | 1730 | 67 | 73 |
| B | 1060 | 1415 | 80 | 72 |
| C | 860 | 1100 | 76 | 73 |

Upon viewing the results of Table II, it is seen that the ROR treatment greatly increased the activity of the cobalt carbonyl catalyst (catalyst A) while providing it with catalyst selectivity (73 weight per cent $C_5$+), comparable to that of the promoted cobalt catalyst B (72 weight per cent $C_5$+) and catalyst C (73 weight per cent $C_5$+). Thus, Tables I and II clearly demonstrate that the combination of an ROR activation treatment of an unpromoted carbonyl catalyst provides a synthesis conversion catalyst having a greatly increased catalyst activity and a catalyst selectivity comparable to promoted cobalt catalysts having a much higher cobalt loading.

Example 2

This example demonstrates the effect of incorporating a promoter into the cobalt carbonyl catalyst of the present invention.

Ruthenium-promoted cobalt carbonyl catalyst samples were prepared by impregnating an alumina support identical to that used in Example 1 with lanthanum nitrate in acetone. The impregnated support was dried to remove the solvent and then calcined for two hours at a temperature of 300 °C. Next, ruthenium acetylacetonate in acetone was added and the catalyst reduced in hydrogen while being heated at a rate of 2 °C per minute until a temperature of 200 °C was reached. The catalyst was then maintained at 200 °C for two hours. This catalyst is denoted "catalyst D". Additional catalyst samples (denoted "catalyst E") were prepared in a manner identical to catalyst D except that the lanthanum nitrate addition step was omitted.

After reduction, the resulting catalyst sample was impregnated with dicobalt octacarbonyl in tetrahydrofuran, without exposure to air, and was stored in a controlled atmosphere glove-box. The catalyst was prepared to contain 12 weight per cent cobalt and 0.3 weight per cent ruthenium in the reduced state.

Next, hydrogen sorption measurements were performed in a static volumetric apparatus, and for each test, 2 gram samples were placed in chemisorption cells inside the glove-box and the cells were then sealed and transferred to the sorption unit. The catalysts were evacuated, then reduced in hydrogen as they were heated to 350 °C and held at that temperature for one hour. After evacuation at 350 °C, the samples were cooled to room temperature for hydrogen sorption measurements. The sorption capacities at 100-500 torr were measured and the total sorption capacity was estimated by extrapolation to zero pressure.

Following the hydrogen sorption capacity measurements, the catalysts were evacuated, then passivated by dosing in air three times to 500 torr and they were then oxidized in flowing air while heating to 300 °C at a rate of 1 °C per minute and were held at that temperature for a period of five and one-half hours. Finally, they were again reduced overnight at 350 °C and their hydrogen sorption capacities were again measured.

The results of the hydrogen sorption and metal disperson tests are set forth below in Table III:

TABLE III

| | | Hydrogen Sorption Capacity | |
| | | (millimoles/gram) | |
| Catalyst | | R350 | ROR |
|---|---|---|---|
| A | | 0.242 | 0.112 |
| D | | 0.234 | 0.169 |
| E | | 0.231 | 0.190 |

| | | Metal Dispersion | |
| | | H/(Co + Ru) | |
| Catalyst | | R350 | ROR |
|---|---|---|---|
| A | | 0.29 | 0.13 |
| D | | 0.27 | 0.22 |
| E | | 0.27 | 0.20 |

Measurements of hydrogen sorption capacities on the slurry catalysts made with dicobalt octacarbonyl showed that their metal dispersions were about 30 per cent after mild reduction. That is about 1.5-2.0 times higher than dispersions obtained by impregnating cobalt nitrate on the same support. Dispersion dropped after ROR treatment, while synthesis activities increased. Activity increase after ROR treatment was not caused by improved metal dispersion, but by an increase in turnover frequency of the metal sites. In other words, the activity per metal site increased indicating that the ROR treatment has brought the catalyst to its most efficient activity per unit weight of metal.

Activity tests were made after similar reduction and ROR treatments, respectively. An 0.5 gram sample of each catalyst was treated in 5,000 cubic centimetres per gram per hour of flowing hydrogen in the reactor before each test. The activity tests were made at atmospheric pressure using 1680 cubic centimetres per gram per hour of synthesis gas flow and 185 °C at a hydrogen to carbon monoxide ratio of 1.85 for a period of 10 to 20 hours onstream. Activity tests were also made at a temperature of 195 °C at a hydrogen to carbon monoxide ratio of 1.5 under atmospheric pressure

- 17 -

and a synthesis gas flow of 1680 cubic centimetres per gram per hour while onstream for 35-45 hours. After activity tests, the catalysts were purged in hydrogen for one hour at 185 °C to strip hydrocarbons, before they were given another activation treatment.

The results are set forth below in Tables IV and V:

## TABLE IV

| Catalyst | CO Conversion Rate (cc/gram Co/hour) | | $C_5+$ Liquid (Wt. %) | |
|---|---|---|---|---|
| | R350 | ROR | R350 | ROR |
| A | 1230 | 1485 | 73 | 81 |
| D | 870 | 2050 | 79 | 80 |
| E | 1010 | 1905 | 67 | 79 |

## TABLE V

| Catalyst | CO Conversion Rate (cc/gram Co/hour) | | $C_5+$ Liquid (Wt. %) | |
|---|---|---|---|---|
| | R350 | ROR | R350 | ROR |
| A | 1525 | 1730 | 67 | 73 |
| D | 1035 | 2170 | 70 | 72 |
| E | 1370 | 2030 | 62 | 71 |

As seen in Table IV, the $C_5+$ selectivity at 185 °C increased from 73 to 81 weight per cent for the unpromoted cobalt on alumina catalyst (A) from 79 to 80 weight per cent for the ruthenium-lanthanum-promoted cobalt catalyst (D) and from 67 to 79 weight per cent for the ruthenium-promoted-cobalt catalyst (E). Thus, these data indicate that the incorporation of ruthenium, alone, or with lanthanum provides a significantly higher catalyst activity using the ROR activation treatment, whereas the selectivity of the unpromoted cobalt catalyst is comparable to that of the promoted cobalt catalyst.

Likewise, similar results are seen in Table V wherein comparable selectivities for $C_5+$ hydrocarbons were achieved for both promoted and unpromoted catalysts. However, catalyst activity was significantly improved using the ruthenium promoter, alone, or in combination with lanthanum.

Example 3

This example demonstrates the effect of using a silica support rather than an alumina support in connection with the cobalt carbonyl catalyst of the present invention.

A catalyst was prepared in the same manner as "Catalyst D", as described in Example 2, with the exception that a fluid silica (commercially available as Ketjen F5) rather than a fluid alumina support was employed. Samples of this catalyst ("Catalyst F") were subjected to activity tests in the same manner as described in Example 2 for catalyst D at 185 °C in a hydrogen to carbon monoxide ratio of 1.85, which test results are set forth in Table VI, below, while the results of tests made at 195 °C at a hydrogen to carbon monoxide ratio of 1.5 are set forth in Table VII, below:

### TABLE VI

| | CO Conversion Rate (cc/gram Co/hour) | | $C_5$+ Liquid (Wt. %) | |
|---|---|---|---|---|
| Catalyst | R350 | ROR | R350 | ROR |
| D | 870 | 2050 | 79 | 80 |
| F | 1074 | 2285 | 62 | 84 |

The results set forth in Table VI show that the use of a silica support provides a higher conversion rate than that achieved with alumina, but with lower $C_5$+ selectivity. However, the $C_5$+ selectivity is much improved by the ROR treatment.

### TABLE VII

| | Co Conversion Rate (cc/gram Co/hour) | | $C_5$+ Liquid (Wt. %) | |
|---|---|---|---|---|
| Catalyst | R350 | ROR | R350 | ROR |
| D | 1035 | 2170 | 70 | 72 |
| F | 1455 | 2730 | 60 | 70 |

Upon viewing the results of Table VII, it is seen once again, that the carbonyl on silica catalyst is more active than the carbonyl on alumina, but at a sacrifice in selectivity. However, the use of a ROR treatment greatly increases the selectivity of the silica supported catalyst.

Example 4

The preparation of the catalysts used in the following experiments is exemplified by the following description of the preparation of the catalyst containing 0.05 weight per cent ruthenium. The support was 70 grams of extrudate of gamma-alumina (Ketjen CK-300 commercially available from Akzo Chemie) which has been ground and sieved to 16-30 mesh size (0.589-1.168 mm) and heated in air at 750 °C for 16 hours. Separate portions comprising 0.1680 gram of ruthenium acetylacetonate, 2.336 grams of lanthanum nitrate, [La(NO$_3$)$_3$ 6H$_2$O], and 87.563 grams of cobalt nitrate, [Co(NO$_3$)$_2$ 6H$_2$O], were dissolved in 181 cubic centimetres of acetone. The solution was divided into three equal parts and the alumina was contacted with the first portion of the catalyst solution with stirring. Solvent was removed from the impregnated alumina in a rotary evaporator at 40 °C. The dried material was then calcined in air at 300 °C for two hours. The calcined catalyst was then impregnated with the second portion of the catalyst solution and the drying and calcining steps were repeated. The calcined catalyst was then impregnated, dried, and calcined as before for a third time. The catalyst analyzed 20.00 weight per cent cobalt, 1.00 weight per cent lanthanum oxide, 0.05 weight per cent ruthenium, and the remainder alumina.

A sample of the catalyst (G) is reduced in 4800 cubic centimetres per gram per hour of hydrogen while heating at the rate of 1 °C per minute to a temperature of 350 °C for a period of fifteen hours. A second sample (H) is reduced by passing 4800 cubic centimetres per gram per hour of hydrogen over the catalyst sample while heating at a temperature of 1 °C per minute until a temperature of 350 °C is reached and then that temperature is maintained for fifteen hours. Next, the reduced catalyst is subjected to passivation and then is oxidized in air at 300 °C for sixteen hours. The oxidized catalyst is then reduced once again by flowing 4800 cubic centimetres per gram per hour of hydrogen while heating at the rate of 1 °C per minute until the temperature of 350 °C is reached and then held for fifteen hours.

The foregoing preparation and activation procedures were repeated, with the exception that the ruthenium content was varied to provide catalyst samples containing 0.10, 0.50 and 1.00 weight per cent ruthenium.

Meanwhile, for comparative purposes, a catalyst substantially identical to the foregoing catalyst was prepared, with the exception that the ruthenium was omitted. The ruthenium-free catalyst was prepared by utilizing a gamma-alumina extrudate (Ketjen C-300) that had been ground and sieved to 16-40 mesh and calcined at 750 °C. An impregnation solution was prepared by dissolving 1.59 grams of lanthanum nitrate, $[La(NO_3)_3 \cdot 6H_2O]$, and 59.28 grams of cobalt nitrate, $[Co(NO_3)_2 \cdot 6H_2O]$, in 120 cubic centimetres of acetone. The impregnation solution was divided into three equal parts and 47.40 grams of the alumina support was saturated with the first portion of the impregnation solution. The solvent was removed in a rotary evaporator at 40 °C, and then calcined in air at 300 °C for two hours. The second portion of impregnation solution is added to the calcined catalyst, evacuated to dryness at 40 °C for one hour and then calcined at 300 °C in air for two hours. The third portion of the catalyst solution is added, and once again, the impregnated catalyst is evacuated to dryness at 40 °C for one hour and calcined at 300 °C in air for two hours. Next, separate samples of the impregnated catalyst are subjected to activation as previously described in connection with the ruthenium-containing catalyst utilizing the type G-H activation treatments.

A series of tests were conducted to evaluate the effect of minute amounts ruthenium as compared with cobalt upon activity of the catalyst in converting synthesis gas to hydrocarbons. In each test, synthesis gas containing 35 weight per cent carbon monoxide and 65 weight per cent hydrogen were passed over the catalyst sample under a pressure of 1 atmosphere. The catalysts activated using procedures G and H were tested using 1680 cubic centimetres of synthesis gas per gram of catalyst per hour.

The results of the tests are set forth in Table VIII below:

TABLE VIII

| | | | | 195 °C | |
|---|---|---|---|---|---|
| | | | | CO Conversion Rate | |
| | | | | (cc/gram metal/hour) | |
| Test No. | (Wt. %) | (Wt.) | (Molar) | R350 | ROR |
| | | | | (G) | (H) |
| 1 | 0.0 | --- | --- | 382 | 476 |
| 2 | 0.05 | 400 | 693 | 780 | 968 |
| 3 | 0.10 | 200 | 346 | 879 | 1093 |
| 4 | 0.50 | 40 | 69 | 1034 | 1415 |
| 5 | 1.00 | 20 | 35 | 930 | 1286 |

As can be seen from the results in Table VIII (Test Nos. 2-5), the use of ruthenium significantly improved catalyst activity as compared with those tests in which no ruthenium was present (Test No. 1). Moreover, of particular significance is the fact that even when the Co/Ru molar ratio exceeded 200/1, namely in Tests 2 and 3, the catalyst activity increased in excess of 100 per cent over that in which ruthenium was absent.

The ROR activation produced catalysts that were 24-34 per cent more active than corresponding catalysts that were reduced just once.

Example 5

The following example demonstrates the improved activity of catalysts activated using the procedure of the present invention when prepared by precipitation.

A catalyst is prepared by aqueous precipitation containing 20 weight per cent cobalt, 1 weight per cent lanthanum oxide with the amount of ruthenium being varied using the procedure described in U.S. Patent No. 4,088,671, in which 125.8 grams of cobalt nitrate,

$[Co(NO_3)_2 \ 6H_2O]$, 1.32 grams of ruthenium chloride, $[RuCl_3]$, and 3.38 grams of lanthanum nitrate, $[La(NO_3)_3 \ 6H_2O]$ were dissolved in 1300 cubic centimetres of distilled water. A second solution was prepared by dissolving 85.5 grams of $K_2CO_3$ in 1300 cubic centimetres of distilled water. The two solutions were separately heated to boiling, and then both solutions were added rapidly with vigorous stirring to 500 cubic centimetres of boiling distilled water, and immediately thereafter 100 grams of 100 mesh gamma-alumina were admixed with stirring and the stirring was continued for 10 minutes. The $K_2CO_3$ coprecipitates the metals as carbonates onto the alumina support. The resultant mixture was filtered rapidly and the precipitate was washed with distilled water until there was no evidence of potassium or nitrates remaining. The precipitate was then dried at 120 °C for 16 hours and then calcined at 350 °C for 16 hours in air. The catalyst was divided into separate portions with the first portion (I) being reduced by passing hydrogen over the catalyst at the rate of 840 cubic centimetres per gram per hour while heating at the rate of 1 °C per minute until the catalyst reached 350 °C at which temperature the catalyst is held for six hours. A separate portion of the catalyst (J) is reduced in hydrogen flowing at the rate of 3500 cubic centimetres per gram per hour while being heated to 110 °C at the rate of 10 °C per minute. The catalyst was held at 110 °C for a period of one hour and then heated to 200 °C at the rate of 0.5 °C per minute, held for two hours, and then heated to 350 °C at the rate of 1 °C per minute and held at 350 °C for 10 hours. Next, passivation of the catalyst is conducted in flowing air, and then the catalyst is reduced once again by passing hydrogen at the rate of 840 cubic centimetres per gram per hour while heating at a rate of 1 °C per minute until the temperature of 350 °C is reached and then holding at that temperature for six hours.

The resulting precipitated catalysts were then tested for activity by contact with synthesis gas containing 2 parts carbon monoxide and 3 parts hydrogen on mole basis at a temperature of 195 °C under 1 atmosphere total pressure at a synthesis gas flow rate of 1680 cc/g/h.

The results are set forth below in Table IX:

TABLE IX

| Test No. | Co (Wt. %) | Ru (Wt. %) | Co/Ru Ratio | | CO Conversion Rate (cc/g metal/h) | |
|---|---|---|---|---|---|---|
| | | | (Wt.) | (Molar) | R350 (I) | ROR (J) |
| 1 | 20 | 0.0 | --- | --- | 210 | 318 |
| 2 | 20 | 0.05 | 400 | 693 | 305 | 414 |
| 3 | 20 | 0.15 | 113 | 231 | 382 | 538 |
| 4 | 20 | 0.50 | 40 | 69 | 563 | 936 |
| 5 | 20 | 1.00 | 20 | 69 | 499 | 477 |

The results set forth in Table IX demonstrate that in all but one case the use of the ROR treatment improved catalyst activity. In tests where the amount of ruthenium exceeded about 0.5 weight per cent, there was a 4% decline.

Example 6

A 20.78 grams portion of Ketjen 000-1:5E gamma alumina was calcined 2 hours at 600 °C, then ground and sieved to 20-40 mesh granules. It was impregnated with 12.34 grams of cobalt nitrate hexahydrate plus 3.68 grams of cerium (III) nitrate hexahydrate dissolved in 24 millilitres of distilled water.

The impregnated catalyst was dried for 2 hours at 120 °C and then was heated in a flow of 200 cc/minute of dry air at 1 °C/minute to 300 °C and was held at that temperature for 2 hours before being cooled and stored. This catalyst is designated as Catalyst K.

A catalyst was prepared in an identical manner, with the same weights of alumina, cerium nitrate, and water, but with 12.33 grams nickel nitrate hexahydrate in place of cobalt nitrate. This catalyst is designated as Catalyst L.

- 24 -

The catalysts were tested for synthesis gas conversion after activation by procedures G (reduction to 350 °C) or H (ROR treatment of the present invention, which is reduction to 350 °C, oxidation to 300 °C, and reduction again to 350 °C). Results obtained at 195 °C, a hydrogen to carbon monoxide molar ratio of 1.5, and atmospheric pressure are set forth in Table X below:

TABLE X

| Catalyst | Wt.% Metal | Wt.% Cerium | CO Conversion Rate (cc/g metal/h) | |
|---|---|---|---|---|
| | | | R350 | ROR |
| K | 10 (Co) | 6 | 650 | 988 |
| L | 10 (Ni) | 6 | 51 | 90 |

The results show that ROR activation also improves the activity of catalysts made by aqueous impregnation, and that it is effective for nickel as well as cobalt.

Example 7

Three of the ruthenium-promoted cobalt catalysts described in Example 4 were also tested for ethane hydrogenolysis after activation procedures G (R350) and H (ROR) of Example 4. The conversions to methane were measured at 225 °C, a hydrogen to ethane molar ratio of 10, and atmospheric pressure. The results are set forth in Table XI below:

TABLE XI

| Test No. | % Co | % Ru | Ethane Conversion Rate (cc/g metal/h) | |
|---|---|---|---|---|
| | | | R350 (F) | ROR (G) |
| 1 | 20 | 0.05 | 16.2 | 24.8 |
| 2 | 20 | 0.10 | 18.8 | 29.6 |
| 3 | 20 | 0.50 | 63.3 | 71.2 |

The results in Table XI show that the ROR activation also improves the catalysts' activity for ethane hydrogenolysis.

C L A I M S

1. Process for the preparation of an activated, supported catalyst comprising depositing a cobalt or nickel precursor on a refractory metal oxide support by impregnation or precipitation to form a supported catalyst, and activating said supported catalyst by subjecting said supported catalyst to the steps, in sequence, of (i) reduction in hydrogen gas, (ii) oxidation in an oxygen-containing gas and (iii) reduction in hydrogen gas, steps (i), (ii) and (iii) being conducted at a temperature of from 100° to 450 °C.

2. Process according to claim 1, wherein a cobalt precursor is used.

3. Process according to claim 2, wherein the cobalt precursor is a cobalt carbonyl compound.

4. Process according to claims 1-3, wherein the refractory metal oxide support is alumina or silica.

5. Process according to claim 4, wherein the refractory metal oxide support is alumina, preferably gamma or eta alumina.

6. Process according to claims 1-5, wherein the cobalt precursor is deposited on the support by impregnating said support with a non-aqueous, organic impregnation solution.

7. Process according to claim 6, wherein the non-aqueous organic impregnation solution is acetone or tetra hydrofuran.

8. Process according to claims 1-7, wherein a ruthenium precursor is deposited on the support, preferably in an amount of 0.05 and 0.50 per cent by weight of the support.

9. Process according to claims 1-8, wherein a lanthanum, manganese or a group IIIB or IVB metal oxide is deposited on the support.

10. Process according to claims 1-9, wherein the first reduction step is conducted at a temperature between 200° and 450 °C, the oxidation step is conducted at a temperature between 100° and 400 °C, and the second oxidation step at a temperature between 200° and 450 °C.

11. Activated supported catalyst prepared according to the process as described in any one of the claims 1-10.

12. Process for the conversion of synthesis gas to a product containing liquid hydrocarbons comprising contacting synthesis gas comprising hydrogen and carbon monoxide under synthesis gas conversion conditions with the activated supported catalyst prepared according to the process of claims 1-10.

13. Hydrocarbons prepared according to the process of claim 12.

14. Process for the preparation of activated, supported catalysts substantially as described hereinbefore with particular reference to the examples.

15. Activated supported catalysts as described hereinbefore with particular reference to the examples.

DNRH04

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | US-A-4 585 798 (H. BEUTHER) * Whole document * | 1-15 | B 01 J 23/74 // B 01 J 23/89 |
| A | US-A-4 492 774 (C.L. KIBBY) | | |
| A | GB-A-1 055 909 (ESSO) | | |
| A | US-A-2 392 107 (J.W. TETER) | | |
| A | GB-A- 576 553 (REVERTEX) * Page 3, example II * | | |
| A | US-A-4 413 064 (H. BEUTHER) | | |

TECHNICAL FIELDS SEARCHED (Int Cl 4)

B 01 J 23/00
B 01 J 37/00

The present search report has been drawn up for all claims

| Place of search | Date of completion of the search | Examiner |
|---|---|---|
| THE HAGUE | 13-04-1987 | DEVISME F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82